(19) Europäisches Patentamt
European Patent Office
Office européen des brevets

(11) **EP 2 290 583 A1**

(12) **EUROPEAN PATENT APPLICATION**
published in accordance with Art. 153(4) EPC

(43) Date of publication:
02.03.2011 Bulletin 2011/09

(51) Int Cl.:
*G06K 9/00* (2006.01)  *A61B 5/117* (2006.01)

(21) Application number: 08874374.5

(22) Date of filing: 06.10.2008

(86) International application number:
**PCT/ES2008/000625**

(87) International publication number:
**WO 2009/141464 (26.11.2009 Gazette 2009/48)**

(84) Designated Contracting States:
AT BE BG CH CY CZ DE DK EE ES FI FR GB GR HR HU IE IS IT LI LT LU LV MC MT NL NO PL PT RO SE SI SK TR
Designated Extension States:
AL BA MK RS

(30) Priority: 23.05.2008 ES 200801530

(71) Applicant: Hanscan IP B.V.
1097JB Amsterdam (NL)

(72) Inventors:
• SANCHEZ DEL RIO SAEZ, Jose
28108 Alcobendas, Madrid (ES)
• RUIZ GARCIA, Antonio, David
28108 Alcobendas, Madrid (ES)

• ANTEQUERA RODRIGUEZ, Nicolas
28108 Alcobendas, Madrid (ES)
• BUENO LOSADA, Roberto
28108 Alcobendas, Madrid (ES)
• MAGUILLO VENEGAS, Sergio, Andres
28108 Alcobendas, Madrid (ES)
• GONZALEZ GONZALEZ, Diego
28108 Alcobendas, Madrid (ES)
• HITA BENITO,Javier
28108 Alcobendas, Madrid (ES)

(74) Representative: Temino Ceniceros, Ignacio
Abril Abogados
Amador de los Rios 1-1°
28010 Madrid (ES)

(54) **METHOD AND BIOMETRIC SCANNER FOR IDENTIFYING A PERSON**

(57) It is based on the fact that the penetration of electromagnetic radiation into the human body depends on the frequency of the incident wave. By lighting the palm of the hand with a plurality of intensity of the same monochromatic light sources, a plurality of vascular maps are obtained.

The hand (2) of the person to be identified is placed on a support (1) that houses an integrated gauge (3) that is equipped with a reflector (4) in the form of a spherical crown, closed at the top by a diffuser (5), inside which there is one upper plate (6) that supports the light sources, and one lower plate (7) that supports a camera (8), whose lens (9) crosses the upper plate (6) and is extended to form a set of filters (10) that cross the diffuser (5).

FIG. 1

## Description

This invention is a procedure for identifying a person, and a biometric scanner for carrying out this procedure. To be more specific, the biometric scanner comprises sources of light and resources for obtaining the vascular map of the palm of a hand of the person to be identified on the basis of a radiation of a pre-established wavelength generated by the light sources, which enables the user to make sure that the person in question is alive, as well as establishing his or her identity (i.e. that the person is who he or she claims to be).

It is to be used in the fields of access control, security for methods of payment, controlling the time employees are at work and, in general, for any purpose that requires a biometric identification of people.

## Background to the invention

Biometric scanners that are based upon acquiring a pattern of veins in the palm of the hand using radiation elements in near infrared radiation (light-emitting diodes with emission wavelength belonging to the infrared close to the electromagnetic spectrum) used as essential elements are well-known, as is the case with radiation receiving elements close to the infrared that enable the user to detect the radiation or to obtain the image concerned from the vascular map of the palm of the hand (also with absorption bands focused on near infrared) and the biological receiver (hand) that interacts with the infrared radiation emitted by the source that emits the light. Thus, the near infrared radiation emitted by the light sources is partly reflected and scattered by the palm of the hand and partly absorbed by the haemoglobin. This radiation, reflected and scattered, contains the information about the vascular map concerning the structure of veins in the palm of the user's hand and is detected by a detection system with an absorption band that is similar to the light emission source absorption band and which enables one to obtain an image of the vein pattern concerned.

Thus, for example, the United States patent application US 2007/0098223 describes a scanner for biometric identification with a low rate of error identification that is based upon reading the vein pattern of the palm of the hand using near infrared radiation. It is an optical gauge that uses as light sources, near infrared radiation emission sources and that uses as detection systems, a CMOS gauge with an absorption spectrum very similar to that of the light source. The infrared radiation emitted by the light sources is reflected and scattered by the epithelial tissue on the palm of the hand and absorbed by the haemoglobin deoxidised in the capillaries. A vein pattern is thus obtained (in the form of an image) that will subsequently be used in a pattern comparison algorithm. The ease with which the vascular map can be replicated once it has been extracted using other methods, entails a serious problem when it comes to usurping the identity of another person.

United States patent US 6813010 describes a gauge that captures the internal pattern of the veins from one single finger by transmitting near infrared radiation through that finger, this patent being equipped with a device for detecting how close the finger is to the aforementioned gauge. The aforementioned detector makes it possible to optimise the intensity of the light that reaches the finger in such a way that a much clearer pattern of the veins is obtained, which facilitates the subsequent recognition process. However, the drawback with this patent lies in the fact that the amount of information that is used for identification purposes is less than in the preceding case.

The application for United States patent US 2007/0116330 describes a device for identifying veins that also makes it possible to detect the presence of living tissue by means of the pattern captured of the roughness of the internal layers of the skin that are covered by epidermal tissue. This roughness pattern is extracted by the difference between the optical properties of the internal layers of the skin and the epidermal tissue. It uses long wavelength radiation such as near infrared radiation that interacts with the skin tissue. Therefore, not only does it record the vein pattern, but it also detects the presence of living tissue. However, the drawback to this invention is that it involves two different processes and two different devices.

The use of near infrared radiation as a means for personal identification for detecting the capillary map of the palm of the hand is a general characteristic of Biometry that is based upon the spectroscopic interaction between the electromagnetic radiation and the epidermis of the hand, and it makes it possible to recognise the user in question through the aforementioned biometric element.

At present, there are very few companies that manufacture gauges that are similar to the ones described, because the manufacturing processes are fraught with major problems. For example, one initial problem revolves around the high manufacturing cost, which renders it unfeasible in certain environments (bank payment terminals), and it also makes it difficult to incorporate it into top-range microcontrollers.

Furthermore, the current error rates in identifying people are really unfavourable when compared to other biometric systems, such as those that are based upon examining the iris. The improvements that have been made with respect to biometric gauges that obtain a vein map have basically been achieved thanks to the development of increasingly sophisticated pattern recognition algorithms, but there have not been any similar improvements in the way the gauges themselves are conceived from a physical perspective.

However, perhaps the greatest problem facing the vein gauges that are currently being used lies in the fact that they can be "deceived", by making false patterns pass through the system, without the user being present.

## Description of the invention

In view of what has been described above, one objective of this invention is to provide a biometric scanner that is extremely reliable when it comes to identifying people.

Another objective of this invention is to provide a biometric scanner that cannot be easily deceived by the system being supplied with vein patterns that have been reproduced on non-living devices.

Finally, it is another objective of this invention to provide a compact and low-cost biometric scanner.

These objectives are achieved with Claim 1, providing a biometric scanner that identifies a person, which is equipped with means for controlling the intensity of the radiation that is generated by the light sources, associated with a power supply loop, in such a way that the resources for obtaining the vascular map obtain different vascular maps of the person to be identified, corresponding to radiations of different intensities emitted by the light sources and determined by the means for controlling the intensity of the radiation.

A biometric scanner is thus obtained that considerably increases the amount of information to be processed, thereby increasing the reliability of the identification, by determining a variety of vascular maps obtained from one single subject. The basis of the invention lies in the fact that the electromagnetic penetration distance in the human body depends on the wavelength of the incident radiation. Once this wavelength has been selected as the working wavelength, this penetration distance also depends upon the intensity emitted by the light sources. In this way, different vascular maps are obtained by lighting up the palm of the hand with light sources that control the intensity associated with a power supply loop, and these vascular maps correspond to the different depths with respect to the surface of the hand on the basis of the intensity emitted.

The two main objectives are thus achieved at the same time, that is to say, an improvement in the reliability of the identification and detecting that the person in question is alive.

In accordance with the realisation of a preferred embodiment of the invention, the radiation that is generated by the light sources is quasi-monochromatic, with a wavelength focused on a range between 720 and 960 nm, which falls within the near infrared absorption band.

As far as the third objective is concerned, obtaining a compact and low-cost device, this is achieved through low-cost emission elements (LEDs) and reception elements (Photodiodes). Furthermore, the above-mentioned feedback mechanism, which makes it possible to control the lighting, takes place through a mathematical algorithm that makes it possible to obtain uniform lighting, even in unfavourable conditions in which there is too much external light (fluorescent lamps, solar radiation, etc.).

This closed loop mechanism for the lighting is described below: in view of the fact that the vein map image that is taken by the gauge depends on the ambience light projected by the objects that surround the gauge, the intensity measured by the CCD/CMOS gauge has two contributions: firstly, light reflected by the hand and the objects coming from the LEDs that are contained inside the vein gauge and, secondly, the ambience light coming from the fluorescent lamps or solar light. A closed loop control is used with a view to stabilising and limiting in three constant thresholds, the amount of infrared radiation that affects the CCD / CMOS gauge at the moment when the image of the hand is being taken. With a view to this, an actuator (controlled potentiometer) regulates the intensity that passes through the vein gauge infrared LEDs on the basis of the quantity of infrared radiation that is detected by the CCD gauge.

It is preferable that the light sources be housed in an integrated gauge that contains the following:

- a cube-shaped reflector, closed at the top with a diffuser;
- an upper plate, where the light sources are arranged;
- a lower plate, which is equipped with a camera that can record the radiation that is emitted by the light sources, whose lens passes through the upper plate, this lens being equipped with an IR transmission filter that passes through the diffuser.

This integrated gauge uses a cube-shaped reflector, closed at the top with a diffuser, which contains both an upper plate and a lower plate. The upper plate supports not only the light sources (which are, for example, light-emitting diodes) but also a set of photo-detectors that are used to detect the presence of the user's hand on the basis of the intensity of the radiation reflected and scattered in the palm of the hand and which makes it possible to initiate the recording procedure for the vascular map of the user's hand. Furthermore, these photodiodes enable the user to know the intensity of radiation that the gauge receives, which together with the intensity detected by the CCD and the intensity thresholds established in the algorithm, makes it possible to find out whether the power supply for the LEDs increases or decreases for cases where the threshold is exceeded or diminished. The lower plate is not only equipped with the required electronics but also a CCD camera, whose optical lens crosses the upper plate with a near infrared filter at the same height as the diffuser.

These light-emitting diodes can be arranged on the upper plate in the form of two concentric rings. In that case, the devices that detect the proximity of the hand of the person to be identified (which are, for example receiving photodiodes), can be arranged layered alternately with respect to the light-emitting diodes, on the upper plate.

Therefore, three lighting thresholds are achieved, on the basis of the intensity detected by the chamber and by the photodiodes, making use of the closed loop.

The elements described should be enough to achieve the proposed objectives. The vein map is hidden and belongs to each user. Falsification is only possible if it is stolen or robbed. To prevent this from happening, it could easily be possible to incorporate, outside the gauge and arranged on a suitable support, one or several more security items in the form of security elements for detecting life, which would make it virtually impossible to sabotage the device, although this would bring about a slight increase in the cost.

A description is given below of these extra security items, which can optionally be selected on the basis of the security and cost requirements specified for a particular application.

First of all, the scanner for this invention could be fitted with a device for detecting the pulse and the oxygen saturation concentration in the blood (the pulse and the oxygen concentration in the blood are characteristics that are inherent to a living person). This device for detecting the pulse and the oxygen saturation concentration in the blood could be equipped with an infrared emitter and an infrared receiver, facing each other through a finger belonging to the person to be identified.

Furthermore, the scanner could also be equipped with a pair of electrodes connected to a device for measuring the bio-impedance and bio-capacity of the hand (human impedance and capacitance are parameters that are inherent to people. They can be used to extract data concerning the user's bodily composition, especially that of the hand that is used in the registration or in the identification. This prevents any other type of element other than human tissue from being used), as well as at least one temperature gauge that is able to detect the temperature of the user's hand (the temperature of the human body is also a characteristic piece of information that is inherent to human beings).

In accordance with another aspect, the invention provides a procedure for identifying a person, which comprises the following stages:

a. Detecting the presence of the hand belonging to the person who is to be identified;
b. Generating an initial radiation from a first specific intensity;
c. Obtaining a first vascular map of the palm of the hand of the person who is to be identified, on the basis of that initial radiation;
d. Generating at least one more radiation from a second specific intensity;
e. Obtaining a second vascular map of the palm of the hand of the person who is to be identified, on the basis of that second radiation;
f. Identifying the person from the first and second vascular maps of the palm of the hand.

**Brief description of the drawings**

A series of drawings are used to make it easier to understand what has been described above, in which a practical embodiment is represented, this being shown merely by way of an example, and is in no way limiting.

In these drawings,

Fig. 1 shows a perspective of the biometric scanner that is the object of the invention, while it is being used;
Fig. 2 shows a close-up in perspective of the extra security items that can be incorporated into the biometric scanner that is the object of the invention;
Fig. 3 shows a vertical section of the integrated gauge;
Fig. 4 shows a plan view of the upper plate of the integrated gauge, by sectioning through Line IV-IV of Figure 3; and

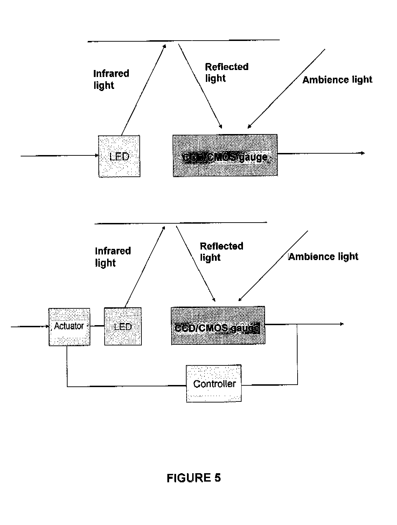

Fig. 5 shows a diagram of the electric power supply loop.

In the figures that have been described, the numbers listed below correspond to the following parts and items:

1. Support
2. User's hand
3. Integrated gauge
4. Cubic reflector
5. Diffuser
6. Upper plate
7. Lower plate
8. CCD camera
9. Camera lens
10. Near infrared filter
11. Photodetectors
12. Wings with pulsioximeter
13. Light-emitting diodes (LEDs)
14. Electrodes
15. Temperature gauge

**Description of a preferred embodiment of the invention**

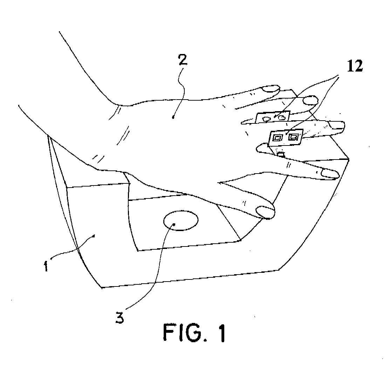

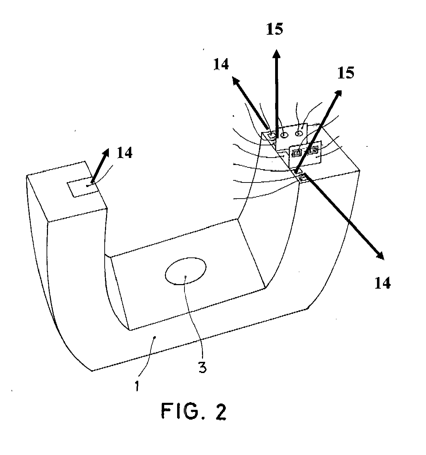

As can be seen in Figures 1 and 2, and in accordance with the invention, the biometric scanner is composed of a support (1) in the form of a "U", on which the hand (2) of the person to be identified is placed. The central part of the support (1) houses an integrated gauge (3), whereas the end branches of the "U" can be used to attach any of the extra security items.

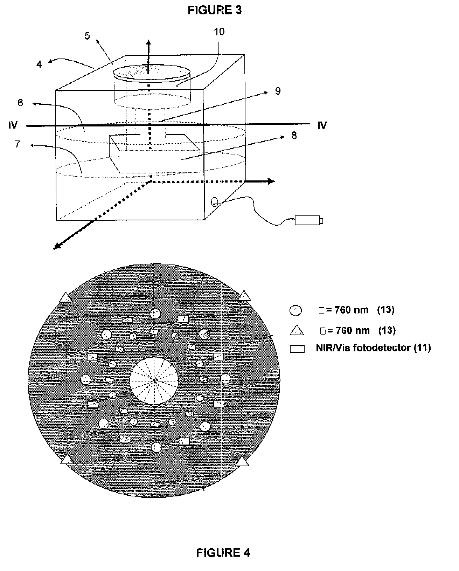

The integrated gauge (3), whose vertical section can be seen in Figure 3, contains a cubic reflector (4), closed by a diffuser (5), inside which two superimposed printed circuit plates are arranged that contain the different components. The light sources and the user proximity detectors are located on the upper plate (6), whereas the lower plate (7) supports not only the electronics that are required but also a camera (8) whose optical lens (9) passes through the upper plate (6) with a filter equipped with a transmission band in the near infrared (10) to remove part of the external visible light that passes through the diffuser (5). This is how the electrical and electronic components are thus separated from the purely optical components, thereby facilitating the possibility of adding new items without the latter interfering with the former.

In this particular preferred embodiment, the diffuser (5) is an opal sheet, whereas the filter (10) allows the infrared radiation to pass within the range of wavelengths in which the light sources of the integrated gauge (3) operate, as well as removing most of the visible component of the spectrum in order to prevent interference to the process of obtaining the vascular maps. The filter (10) is also equipped with a mobile polariser with a rotating polarisation axis.

The camera (8) is of the Charge-Coupled Device (CCD) type and is equipped with an optical range that, apart from picking up the vascular maps with the infrared, enables the user to take an image of the silhouette of the hand to make sure that it is in the right position.

The way that the light sources on the upper plate (6) are arranged can be seen in Figure 3. There are a series of light-emitting diodes (LED) with an emission wavelength of 880 nm in the form of two concentric rings, between which receiving photodiodes are inserted. The corners of the upper plate (6) are also equipped with these diodes.

The extra security items are grouped around the two branches of the "U" that constitutes the scanner support (1), as can be seen in Figure 2.

The device for reading the pulse and the oxygen saturation concentration in the blood is equipped with two wings (12) that, in turn, are arranged on the far right of the support (1). This detector is equipped with an infrared emitter consisting of two light-emitting diodes with wavelengths of 660 nm and 940 nm, which correspond to the oxidised haemoglobin and deoxidised haemoglobin peaks, and an infrared receiver, facing the infrared emitter, which contains a photodetector that has an absorption band within the aforementioned range.

The electrical properties (impedance and capacitance) of the hand (2) are measured by means of electrodes (14) that are located on the ends of the "U" that constitutes the scanner support (1).

Furthermore, two temperature gauges (15), positioned on either side of the wings (12) of the support (1), enable the user to take the temperature of two fingers on the hand (2).

Figure 4 is a diagram that shows how the lighting system with the closed loop operates to provide standard and constant lighting. It also enables the operator to level the lighting at a constant threshold, so that a clear image can be obtained in any outdoor environment.

The way the biometric scanner that is the object of the invention works is as follows:

• *Characterisation and calibration*

The integrated gauge can be used for the spectral calibration of the CCD camera before the scanner is installed where it is to be used. The outlet hole for the radiation from the integrated gauge gives a specific directionality for the emitting source. The emission takes place vertically from the bottom to the top. The hand placed on the gauge (see Figure 1) protects the CCD camera from the ambience light sources, whose emission direction is mainly from top to bottom. The main radiation component that is thus propagated vertically in a downward direction is due to the radiation that is reflected on the palm of the hand and which provides the required information, rather than the radiation due to environmental sources.

• *Proximity detection in stroboscopic operation*

The scanner is usually in this operating mode, in which all the diodes are activated intermittently. When the user's hand (2) approaches, the radiation reemitted after being absorbed by the user's veins is detected. Prior to this presence detection stage.

• *Lighting control*

The actuator (potentiometer controlled by the controller) begins to regulate the LED power input, on the basis of the average colour threshold of the pixels on the image detected by the CCD, until a threshold is reached that has been programmed to achieve a first desired intensity. This average threshold for the image is also obtained from the radiation detected by the photodiodes (11) located on the lower plate containing *LEDs* and, on the basis of the intensity of the signal received, the controller sends again the order to either increase or decrease the power for the LEDs, while at the same time making a comparison with the average pixel information detected by the CCD. This process continues until two different intensity levels (and thus two lighting levels) are reached. One characteristic vein map of the user's hand is extracted for each threshold that is reached. Therefore, three vein maps are used in the biometric system described above.

As far as the industrial realisation is concerned, it will be clear to an expert in the subject matter that a series of modifications and options can be made to adapt the realisation to specific applications. It is thus possible to make the following considerations about the design.

• *The receiving element that the scanner is equipped with and its biometric features*

The biometric interaction element in a biometric gauge is a biological receiver, which interacts physically with the measurable physical magnitude, characteristic of the type of biometric gauge that is used. This biometric element possesses biological characteristics that are unique to the individual concerned, (biometric characteristics), which have to be detected by the detection system so that they can subsequently be processed. The biometric features are generally within a biological receiver. In view of the fact that the gauge that is the object of the invention uses the optical principle of interaction between IR radiation and the palm of the hand, it is an optical biometric system in which the palm of the hand is a receiving biological element. Its biometric characteristics are capillaries through which the blood flows (oxidised and deoxidised haemoglobin). Therefore, the system that processes or treats the signal has to extract these biometric features (the capillary map of the hand) from the receiving element (the hand).

As has already been pointed out, haemoglobin is the biological substance that plays the most important role in the biological recognition carried out by the gauge that has been developed. Therefore, it is essential to be very familiar with the haemoglobin's maximum absorption peaks with the electromagnetic radiation in order to be able to establish which types of light sources have to be used. There are several of these peaks, depending on whether or not the haemoglobin is oxidised in the blood. The maximum peak is $\lambda$=760 nm for deoxidised haemoglobin, although there are many others, most of which are centred on the IR (near and far). There is also a maximum peak for visible red light ($\lambda$=650 nm). Therefore, haemoglobin has a very broad absorption band within the electromagnetic spectrum IR range.

• *The light sources*

In view of the fact that the haemoglobin absorption band spreads mainly in the IR zone of the spectrum, plus the fact that the electromagnetic radiation light-emitting sources focused on the near IR are low cost, light-emitting diodes (LEDs) have been used with wavelengths centres on 880 nm.

• *The detection system*

On the basis of the wavelength for the light sources chosen, the receiver selected is a CCD camera that is based upon silicon microelectronics technology. Other radiation detectors are possible.

• *The optical filters*

As has already been pointed out, the camera (8) is sensitive not only to near IR but also to the visible range, so it is necessary to use light filters with a high transmittance level in the IR and almost zero in the visible. As an alternative to the near infrared optical filters, and as a lower cost option measure, two linear polarising sheets can be used, that are crossed in the visible range and that are located (as is the case for the optical filters) on the lens (9) of the camera (8).

• *The CCD camera lens system*

With a view to obtaining an optimum image formation in the CCD camera and greater integration, it is advisable to use a lens (9) with a wide angle (angle range of 180°) with a focusing distance of f=1.6 mm.

• *Extra security elements*

The mathematical model for the pulsioximeter is based upon measuring the time taken for the light intensity to pass through a fine tissue, such as for example the tip of the finger. The signal process is based upon this simple model and upon the *Beer-Lambert Law:*

$$I(\lambda,t) = I_o(\lambda).e^{-(\sum_v (\sigma_{Hb_v}(\lambda) s_{Hb_v}))z(t)}$$

where the intensity $I_0$ and $\lambda$ is the wavelength of the incident light, $c_{Hb}(\lambda)$ is the concentration and $s_{Hb}(\lambda)$ is the coefficient of absorption of the wavelength depending on each derivate of Hb; z describes the variable thickness. Its realisation would not cause any problems to an expert in the subject matter.

Before the lighting control process for the subsequent plotting of the vein maps of the user's palm, the pulsioximeter detects the oxygen saturation concentration in the blood and the user's pulse. If the values detected are beyond the characteristic threshold for humans, the gauge does not carry on plotting the vein maps and tells the user to position the hand correctly on the gauge.

The electrical properties (impedance and capacitance) of the hand tissues are advantageously measured by the electrodes (14), by means of an alternating current of 800 microamperes and 50 kHz.

In spite of having described and represented a specific realisation of this invention, it is clear that an expert in the subject matter will be able to make variations and modifications, or replace the details with other that are technically equivalent, without moving away from the sphere of protection defined in the enclosed claims.

Furthermore, in spite of the fact that the realizations described for the invention with reference to the drawings include counting systems and processes that take place in counting systems (for example, to obtain the vascular maps), the invention also applies to computer programs, especially to computer programs in or on portability resources that are adapted for the purpose of implementing the invention. The computer program can be either in the form of a source code or an object code, or in an intermediate code between source code and object code, or in partially compiled form, or in any other form that is suitable for use in implementing the processes in a way that is consistent with the invention. The portability resource can be any device that is capable of supporting the program.

For example, the portability medium could be a storage facility, such as a *ROM*, for example a *CD ROM* or a semi-conducting *ROM*, or a magnetic recording medium, for example a floppy disk or a hard disk. Furthermore, the supporting medium could be one that can be transmitted, such as an electrical or optical signal that can be transmitted through an electrical or optical cable or by radio or other means.

When the computer program is contained in a signal that can be transmitted directly via a cable or another device or medium, the medium in question can be composed of that cable or another device or medium.

As an alternative, the portability medium could be an integrated circuit in which the computer program is embedded, the integrated circuit being adapted to carry out the required processes, or to be used in relation with such processes.

## Claims

1. A biometric scanner to identify a person, which is equipped with light sources and resources for obtaining a vascular map of a palm of the hand (2) of the person to be identified, by means of the radiation of a pre-established wavelength generated by the light sources, **characterised by** the fact that it also has resources for controlling the intensity of the radiation that is generated by the light sources, associated with a power supply loop, in such a way that the means for obtaining the vascular map obtain different vascular maps of the person to be identified, corresponding to radiations of different intensities emitted by the light sources and determined by the resources for controlling the intensity of the radiation.

2. Scanner as in Claim 1, **characterised by** the fact that the radiation generated by the light sources is quasi-monochromatic with the wavelength centred on a range between 720 and 960 nm, which lies within the absorption band of the near infrared.

3. Scanner as in any of Claims 1 or 2, **characterised by** the fact that the light sources are housed in an integrated gauge (3) that is composed of:

   - a reflector (4) in the form of a cube, closed at the top by a diffuser (5);
   - an upper plate (6), which contains the light sources;
   - a lower plate (7), which is equipped with a camera (8) that can record the radiation that is emitted by the light sources, its lens (9) passing through the upper plate (6), this lens being equipped with an IR transmission filter (10) that passes through the diffuser (5).

4. Scanner as in any of Claims 1 to 3, **characterised by** the fact that the light sources are light-emitting diodes.

5. Scanner as in Claim 4, **characterised by** the fact that the light-emitting diodes are arranged in the form of two concentric rings in the upper plate (6).

6. Scanner as in any of Claims 3 to 5, **characterised by** the fact that the upper plate (6) is also equipped with devices for detecting the proximity of the hand belonging to the person to be identified.

7. Scanner as in Claim 6, **characterised by** the fact that the proximity detectors are receiving photodiodes (13).

8. Scanner as in Claim 7, **characterised by** the fact that the receiving photodiodes (13) are inserted alternately with respect to the light-emitting diodes on the upper plate (6).

9. Scanner as in any of Claims 7 or 8, **characterised by** the fact that, on the basis of the intensity that is detected by the camera (8) and by the photodiodes (13), three lighting thresholds are achieved, making use of the closed loop.

10. Scanner as in any of preceding claim, **characterised by** the fact that it is equipped with a device for reading the pulse and detecting the concentration of oxygen saturation in the blood (12).

11. Scanner as in Claim 10, **characterised by** the fact that the device for reading the pulse and detecting the concentration of oxygen saturation in the blood is equipped with an infrared emitter and an infrared receiver (12), facing each other through a finger of the hand (2) belonging to the person to be identified.

12. Scanner as in any of preceding claims, **characterised by** the fact that it has a pair of electrodes (14) connected to a device for measuring the bio-impedance and bio-capacitance of the hand (2).

13. Scanner as in any of the preceding claims, **characterised by** the fact that it is equipped with at least one temperature gauge (15) that can measure the temperature of the user's hand (2).

14. A procedure for identifying a person, which comprises the following stages:

   a. Detecting the presence of the hand belonging to the person who is to be identified;

**EP 2 290 583 A1**

b. Generating an initial radiation of a first specific intensity;

c. Obtaining a first vascular map of the palm of the hand (2) of the person who is to be identified, on the basis of that initial radiation;

d. Generating at least one more radiation of a second specific intensity;

e. Obtaining a second vascular map of the palm of the hand (2) of the person who is to be identified, on the basis of that second radiation;

f. Identifying the person from the first and second vascular maps of the palm of the hand (2).

FIG. 1

FIG. 2

FIGURE 3

FIGURE 4

Infrared
light

Reflected
light

Ambience light

LED

CCD/CMOS gauge

Infrared
light

Reflected
light

Ambience light

Actuator

LED

CCD/CMOS gauge

Controller

FIGURE 5

# INTERNATIONAL SEARCH REPORT

| International application No. |
| --- |
| PCT/ ES 2008/000625 |

**A. CLASSIFICATION OF SUBJECT MATTER**

see extra sheet

According to International Patent Classification (IPC) or to both national classification and IPC

**B. FIELDS SEARCHED**

Minimum documentation searched (classification system followed by classification symbols)
G06K, A61B

Documentation searched other than minimum documentation to the extent that such documents are included in the fields searched

Electronic data base consulted during the international search (name of data base and, where practicable, search terms used)

INVENES, EPODOC, WPI, TXTE

**C. DOCUMENTS CONSIDERED TO BE  RELEVANT**

| Category* | Citation of document, with indication, where appropriate, of the relevant passages | Relevant to  claim No. |
| --- | --- | --- |
| Y | US 2006110145 A1 (FUJIMOTO et al.) 25.05.2006, abstract; paragraphs [1-17]; paragraphs [40-60]; paragraphs [85-103]; figures 1-5 and 13-15. | 1-14 |
| Y | US 2006062438 A1 (ROWE) 23.03.2006, abstract; paragraphs [6-8]; paragraphs [46-49]; figures 1A and 4. | 1-14 |
| Y<br>A | US 2007206098 A1 (MATSUO et al.) 06.09.2007, abstract; paragraphs [6-17]; paragraphs [63-112]; figures 1-10 and 16-22. | 6-9<br>3,5 |
| Y | US 2007021661 A1 (DELONZOR et al.) 25.01.2007, abstract; paragraphs [2-3]; paragraphs [48-49]; figures 9-10. | 10,11 |
| Y<br>A | EP 1708135 A1 (FUJITSU LTD) 04.10.2006, abstract; column 5, lines 11-16 and lines 36-39; paragraphs [51-55]; paragraphs [72-75]; figures 3 and 6. | 12<br>10 |

☒ Further documents are listed in the continuation of Box C.          ☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance.<br>"E" earlier document but published on or after the international filing date | |
| "L" document which may throw doubts on priority claim(s) or which is cited to establish the publication date of another citation or other special reason (as specified)<br>"O" document referring to an oral disclosure use, exhibition, or other means<br>"P" document published prior to the international filing date but later than the priority date claimed | "X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other documents , such combination being obvious to a person skilled in the art |
| | "&" document member of the same patent family |

| Date of the actual completion of the international search | Date of mailing of the international search report |
| --- | --- |
| 16.February.2009          (16.02.2009) | **(23/02/2009)** |
| Name and mailing address of the ISA/<br>O.E.P.M.<br><br>Paseo de la Castellana, 75 28071 Madrid, España.<br>Facsimile No.   34 91 3495304 | Authorized officer<br>       A. Figuera González<br><br>Telephone No. +34 91 349 55 16 |

Form PCT/ISA/210 (second sheet) (July 2008)

## INTERNATIONAL SEARCH REPORT

| | International application No. |
|---|---|
| | PCT/ES 2008/000625 |

| C (continuation). | DOCUMENTS CONSIDERED TO BE   RELEVANT |
|---|---|

| Category* | Citation of documents, with indication, where appropriate, of the relevant passages | Relevant to claim No. |
|---|---|---|
| Y | EP 1739592 A1 (FUJITSU LTD ; FUJITSU FRONTECH LTD) 03.01.2007, abstract; paragraphs [35-37]; figure 3, | 13 |

Form PCT/ISA/210 (continuation of second sheet) (July 2008)

## INTERNATIONAL SEARCH REPORT

Information on patent family members

| | International application No. |
|---|---|
| | PCT/ ES 2008/000625 |

| Patent document cited in the search report | Publication date | Patent family member(s) | Publication date |
|---|---|---|---|
| US 2006110145 A | 25.05.2006 | WO 2004088588 A | 14.10.2004 |
| | | AU 2003220951 A | 25.10.2004 |
| | | CN 1689038 A | 26.10.2005 |
| | | CN 100395775 C | 18.06.2008 |
| | | EP 1610265 A | 28.12.2005 |
| | | US 7415202 B | 19.08.2008 |
| US 2006062438 A | 23.03.2006 | CA 2521304 A | 21.10.2004 |
| | | AU 2004227886 A | 21.10.2004 |
| | | WO 2004090786 A | 21.10.2004 |
| | | US 2004240712 A | 02.12.2004 |
| | | US 7147153 B | 12.12.2006 |
| | | US 2005205667 A | 22.09.2005 |
| | | US 7347365 B | 25.03.2008 |
| | | US 2005265585 A | 01.12.2005 |
| | | US 2005265586 A | 01.12.2005 |
| | | US 7394919 B | 01.07.2008 |
| | | US 2005271258 A | 08.12.2005 |
| | | US 7460696 B | 02.12.2008 |
| | | EP 1611541 A | 04.01.2006 |
| | | US 2006002597 A | 05.01.2006 |
| | | US 7440597 B | 21.10.2008 |
| | | US 2006002598 A | 05.01.2006 |
| | | US 7386152 B | 10.06.2008 |
| | | KR 20060002923 A | 09.01.2006 |
| | | US 2006110015 A | 25.05.2006 |
| | | CA 2593007 A | 13.07.2006 |
| | | WO 2006074407 A | 13.07.2006 |
| | | AU 2006203899 A | 13.07.2006 |
| | | WO 2006093508 A | 08.09.2006 |
| | | CA 2569440 A | 08.09.2006 |
| | | AU 2005328364 A | 08.09.2006 |
| | | US 2006202028 A | 14.09.2006 |
| | | US 2006210120 A | 21.09.2006 |
| | | US 2006274921 A | 07.12.2006 |
| | | US 2007030475 A | 08.02.2007 |
| | | CA 2621379 A | 08.03.2007 |
| | | US 2007116331 A | 24.05.2007 |
| | | EP 1789908 A | 30.05.2007 |
| | | EP 20050857436 | 26.05.2005 |
| | | JP 2007524441 T | 30.08.2007 |
| | | EP 1839233 A | 03.10.2007 |
| | | EP 20060733642 | 05.01.2006 |
| | | KR 20070103753 A | 24.10.2007 |
| | | JP 2008501196 T | 17.01.2008 |
| | | CN 101124588 A | 13.02.2008 |
| | | CN 101194270 A | 04.06.2008 |
| | | JP 2008527544 T | 24.07.2008 |
| | | US 2008298649 A | 04.12.2008 |
| US 2007206098 A | 06.09.2007 | CN 101030245 A | 05.09.2007 |

Form PCT/ISA/210 (patent family annex) (July 2008)

**EP 2 290 583 A1**

## INTERNATIONAL SEARCH REPORT
Information on patent family members

| | | International application No. |
|---|---|---|
| | | PCT/ ES 2008/000625 |

| Patent document cited in the search report | Publication date | Patent family member(s) | Publication date |
|---|---|---|---|
| | | EP 1830305 A | 05.09.2007 |
| | | KR 20070090725 A | 06.09.2007 |
| | | JP 2007229360 A | 13.09.2007 |
| US 2007021661 A | 25.01.2007 | US 5797841 A | 25.08.1998 |
| | | US 6173196 B | 09.01.2001 |
| | | US 2001000790 A | 03.05.2001 |
| | | US 6430423 B | 06.08.2002 |
| | | US 2002173708 A | 21.11.2002 |
| | | US 6763255 B | 13.07.2004 |
| | | US 2007015982 A | 18.01.2007 |
| | | US 7389130 B | 17.06.2008 |
| | | US 2007021659 A | 25.01.2007 |
| | | US 7373188 B | 13.05.2008 |
| | | US 2007021660 A | 25.01.2007 |
| | | US 7373189 B | 13.05.2008 |
| | | US 7386334 B | 10.06.2008 |
| | | US 2007021663 A | 25.01.2007 |
| | | US 7321790 B | 22.01.2008 |
| | | US 2007021662 A | 25.01.2007 |
| | | US 7373191 B | 13.05.2008 |
| | | US 2007027378 A | 01.02.2007 |
| | | US 7369886 B | 06.05.2008 |
| | | US 2007027377 A | 01.02.2007 |
| | | US 7418284 B | 26.08.2008 |
| | | US 2007027380 A | 01.02.2007 |
| | | US 2007027379 A | 01.02.2007 |
| | | US 7373190 B | 13.05.2008 |
| | | US 7190984 B | 13.03.2007 |
| EP 1708135 A | 04.10.2006 | WO 2005069212 A | 28.07.2005 |
| EP 1739592 A | 03.01.2007 | EP 20060250656 | 07.02.2006 |
| | | US 2007003112 A | 04.01.2007 |
| | | KR 20070003533 A | 05.01.2007 |
| | | CN 1892679 A | 10.01.2007 |
| | | JP 2007011769 A | 18.01.2007 |

Form PCT/ISA/210 (patent family annex) (July 2008)

16

## INTERNATIONAL SEARCH REPORT

International application No.

PCT/ ES 2008/000625

CLASSIFICATION OF SUBJECT MATTER

*G06K 9/00* (2006.01)
*A61B 5/117* (2006.01)

Form PCT/ISA/210 (extra sheeet) (July 2008)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070098223 A
- US 6813010 B
- US 20070116330 A